# EUROPEAN PATENT APPLICATION

(11) **EP 4 098 262 A1**
(43) Date of publication of application: **07.12.2022**
(21) Application number: 21768861.3
(22) Date of filing: 10.03.2021
(51) Int. Cl.: A61K 31/506, A61K 31/4985, A61K 31/495, A61P 3/04, A61P 3/06

(54) **PHARMACEUTICAL COMPOSITION FOR PREVENTION OR TREATMENT OF DIABETES AND METABOLIC DISEASES ASSOCIATED THEREWITH**

(30) Priority: 11.03.2020 KR 20200030424
(71) Applicant: Dong-A ST Co., Ltd., Dongdaemun-gu Seoul 02587 (KR)
(72) Inventor: KIM, Mi-Kyung, Suwon-Si Gyeonggi-do 16505 (KR); KIM, Tae Hyoung, Seoul 05501 (KR); JUNG, Il Hoon, Anyang-si Gyeonggi-do 13994 (KR); CHAE, Yu Na, Yongin-Si Gyeonggi-do 16807 (KR); YANG, Jae Sung, Seoul 06624 (KR)
(74) Representative: Gille Hrabal Partnerschaftsgesellschaft mbB Patentanwälte
(86) International application number: PCT/KR2021/002990
(87) International publication number: WO 2021/182877

(57) **Abstract**

The present invention relates to a pharmaceutical composite composition for prevention or treatment of diabetes mellitus and at least one disease selected from metabolic diseases associated therewith. The pharmaceutical composition according to the present invention exhibits excellent effects of lowering blood glucose levels, body weight, and blood lipid levels. Therefore, the composition can be advantageously used for preventing or treating diabetes mellitus and at least one disease selected from metabolic diseases associated therewith.

## Description

### Technical Field

The present invention relates to a pharmaceutical composition for prevention or treatment of diabetes and metabolic diseases associated therewith, containing a G protein coupled receptor 119 (GPR119) ligand and at least one drug of another mechanism as an active ingredient.

### Background

Diabetes is a chronic progressive disease characterized by hyperglycemia caused by multifactorial factors. Thus, when individual drugs are used alone, there are frequent cases in which sufficient effects of preventing or treating diabetes are not achieved depending on symptoms. For this reason, although more than a dozen drugs with different mechanisms have already come into the market, there is a continuous need for the development of hypoglycemic agents with various mechanisms of action.

Meanwhile, metabolic diseases associated with diabetes are a problem among diabetic patients. Specifically, obesity or dyslipidemia associated with diabetes is a problem.

### Detailed Description of the Invention

### Technical Problem

One object of the present invention is to provide a pharmaceutical composition capable of alleviating postprandial and fasting blood glucose level when administered to a subject.

One object of the present invention is to provide a pharmaceutical composition exhibiting an effect of alleviating a blood lipid concentration and reducing body fat when administered to a subject.

One object of the present invention is to provide a pharmaceutical composition which can be advantageously used for preventing or treating diabetes or metabolic diseases associated therewith.

### Technical Solution

A pharmaceutical composition for preventing or treating diabetes or at least one disease selected from metabolic diseases associated therewith according to one embodiment of the present invention may include a compound represented by a following Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor.

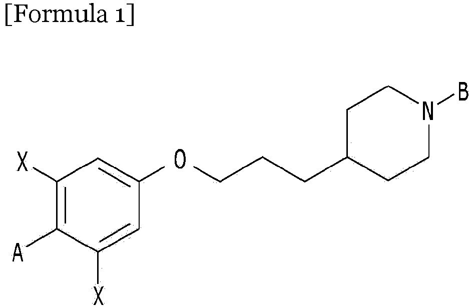

In above Formula 1, A may be an oxadiazole group, a dihydrooxazole group, a thiazole group or a thiadiazole group. Above A may be unsubstituted or substituted with at least one substituent selected from the group consisting of a halogen atom, a C1-C6 straight or branched chain alkyl group and a C1-C6 straight or branched chain hydroxyalkyl group. The alkyl group or the hydroxyalkyl group may be each independently unsubstituted or substituted with a halogen atom or a C1-C6 alkoxy group.

B may be a pyridine group, a pyrimidine group, a pyrazine group or an oxadiazole group. Above B may be unsubstituted or substituted with at least one substituent selected from the group consisting of a halogen atom, a C1-C6 straight or branched chain alkyl group, a C1-C6 straight or branched chain hydroxyalkyl group, a C1-C6 alkoxy group and an oxadiazole group. The C1-C6 straight or branched chain alkyl group, the C1-C6 straight or branched chain hydroxyalkyl group, the C1-C6 alkoxy group or the oxadiazole group may be each independently unsubstituted or substituted with halogen, a C1-C6 alkyl group or a C1-C6 alkoxy group.

X₁ and X₂ may be F, Cl, Br or I. X₁ and X₂ may be the same or different from each other.

Above A may be

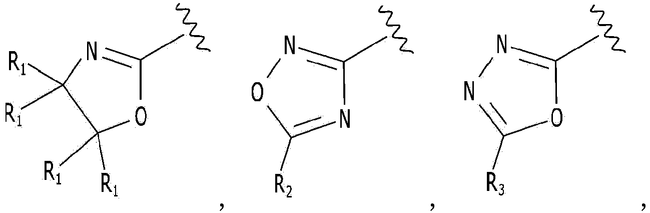

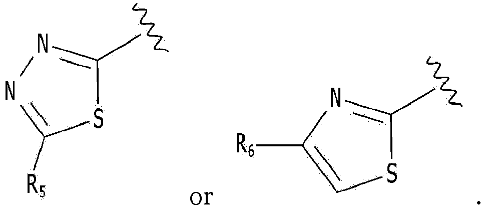

R₁ to R₃, R₅, and R₆ may be each independently at least one substituent selected from the group consisting of a hydrogen atom, a halogen atom, a C1-C6 straight or branched chain alkyl group and a C1-C6 straight or branched chain hydroxyalkyl group. The alkyl group or the hydroxyalkyl group may be each independently unsubstituted or substituted with a halogen atom or a C1-C6 alkoxy group.

Above B may be

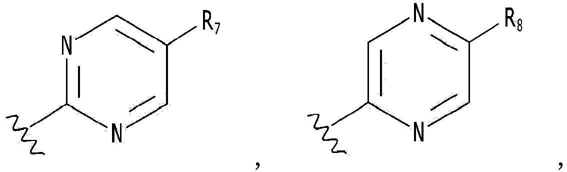

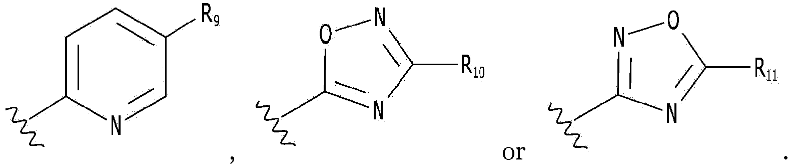

Above R₇ to R₁₁ may be each independently substituted with at least one substituent selected from the group consisting of a hydrogen atom, a halogen atom, a C1-C6 straight or branched chain alkyl group, a C1-C6 straight or branched chain hydroxyalkyl group, a C1-C6 alkoxy group and an oxadiazole group. The alkyl group, the hydroxyalkyl group, the alkoxy group or the oxadiazole group may be each independently unsubstituted or substituted with a halogen atom, a C1-C6 alkyl group or a C1-C6 alkoxy group.

X may be F.

In above formula 1, above A may be an oxadiazole group substituted with Ci-C6 straight or branched chain alkyl group, above B may be a pyrimidine group substituted with C1-C6 straight or branched chain alkyl group, and X may be F.

In the present embodiment, the term "halogen" may refer to fluorine, chlorine, bromine or iodine.

In one embodiment, the term "alkyl" may refer to a straight or branched chain hydrocarbon residue unless otherwise specified. An example of said C1-C6 alkyl may include methyl, ethyl, propyl, isopropyl, butyl, isobutyl, pentyl, hexyl, etc.

In one embodiment, the term "alkoxy" may include an alkyl-oxygen radical having alkyl as defined above unless otherwise specified. An example of said C1-C6 alkoxy may include methoxy, ethoxy, propoxy, butoxy, pentoxy, etc.

In one embodiment, the term "heterocycle" or "heterocyclic" may refer to a 5 to 13-membered hetero-aromatic or non-aromatic compound including 1 to 3 heteroatoms selected from the group consisting of N, O, and S unless otherwise specified.

In one embodiment, the compound represented by above Formula 1 may be at least one compound specifically selected from the group consisting of the following compounds:
2-(4-(3-(1-(5-ethylpyrimidin-2-yl)piperidin-4-yl)propoxy)-2,6-difluorophenyl)-4,5-dihydrooxazole,
(R)-2-(4-(3-(1-(5-ethylpyrimidin-2-yl)piperidin-4-yl)propoxy)-2,6-difluorophenyl)-4-methyl-4,5-dihydrooxazole,
(S)-2-(4-(3-(1-(5-ethylpyrimidin-2-yl)piperidin-4-yl)propoxy)-2,6-difluorophenyl)-4-methyl-4,5-dihydrooxazole,
(S)-2-(4-(3-(1-(5-ethylpyrimidin-2-yl)piperidin-4-yl)propoxy)-2,6-difluorophenyl)-5-methyl-4,5-dihydrooxazole,
(R)-2-(4-(3-(1-(5-ethylpyrimidin-2-yl)piperidin-4-yl)propoxy)-2,6-difluorophenyl)-5-methyl-4,5-dihydrooxazole,
2-(4-(3-(1-(5-ethylpyrimidin-2-yl)piperidin-4-yl)propoxy)-2,6-difluorophenyl)-5,5-dimethyl-4,5-dihydrooxazole,
(R)-(2-(4-(3-(1-(5-ethylpyrimidin-2-yl)piperidin-4-yl)propoxy)-2,6-difluorophenyl)-4,5-dihydrooxazol-5-yl)methanol,
(S)-(2-(4-(3-(1-(5-ethylpyrimidin-2-yl)piperidin-4-yl)propoxy)-2,6-difluorophenyl)-4,5-dihydrooxazol-5-yl)methanol,
(R)-3-(2-(4-(3-(3,5-difluoro-4-(5-methyl-4,5-dihydrooxazol-2-yl)phenoxy)propyl)piperidin-1-yl)pyrimidin-5-yl)-5-isobutyl-1,2,4-oxadiazole,
(R)-5-(4-(3-(3,5-difluoro-4-(4-methyl-4,5-dihydrooxazol-2-yl)phenoxy)propyl)piperidin-1-yl)-3-isopropyl-1,2,4-oxadiazole,
(S)-5-(4-(3-(3,5-difluoro-4-(5-methyl-4,5-dihydrooxazol-2-yl)phenoxy)propyl)piperidin-1-yl)-3-isopropyl-1,2,4-oxadiazole,
5-(4-(3-(4-(5,5-dimethyl-4,5-dihydrooxazol-2-yl)-3,5-difluorophenoxy)propyl)piperidin-1-yl)-3-isopropyl-1,2,4-oxadiazole,
3-(4-(3-(1-(5-ethylpyrimidin-2-yl)piperidin-4-yl)propoxy)-2,6-difluorophenyl)-5-methyl-1,2,4-oxadiazole,
3-(4-(3-(1-(5-ethylpyrimidin-2-yl)piperidin-4-yl)propoxy)-2,6-difluorophenyl)-5-propyl-1,2,4-oxadiazole,
3-(4-(3-(1-(5-ethylpyrimidin-2-yl)piperidin-4-yl)propoxy)-2,6-difluorophenyl)-5-isopropyl-1,2,4-oxadiazole,
5-(tert-butyl)-3-(4-(3-(1-(5-ethylpyrimidin-2-yl)piperidin-4-yl)propoxy)-2,6-difluorophenyl)-1,2,4-oxadiazole,
(3-(4-(3-(1-(5-ethylpyrimidin-2-yl)piperidin-4-yl)propoxy)-2,6-difluorophenyl)-1,2,4-oxadiazol-5-yl)methanol,
2-(3-(4-(3-(1-(5-ethylpyrimidin-2-yl)piperidin-4-yl)propoxy)-2,6-difluorophenyl)-1,2,4-oxadiazol-5-yl)ethan-1-ol,
(S)-1-(3-(4-(3-(1-(5-ethylpyrimidin-2-yl)piperidin-4-yl)propoxy)-2,6-difluorophenyl)-1,2,4-oxadiazol-5-yl)propan-1-ol,
(R)-1-(3-(4-(3-(1-(5-ethylpyrimidin-2-yl)piperidin-4-yl)propoxy)-2,6-difluorophenyl)-1,2,4-oxadiazol-5-yl)propan-2-ol,
(S)-1-(3-(4-(3-(1-(5-ethylpyrimidin-2-yl)piperidin-4-yl)propoxy)-2,6-difluorophenyl)-1,2,4-oxadiazol-5-yl)propan-2-ol,
2-(3-(4-(3-(1-(5-ethylpyrimidin-2-yl)piperidin-4-yl)propoxy)-2,6-difluorophenyl)-1,2,4-oxadiazol-5-yl)-2-methylpropan-1-ol,
3-(2,6-difluoro-4-(3-(1-(5-propylpyrimidin-2-yl)piperidin-4-yl)propoxy)phenyl)-5-isopropyl-1,2,4-oxadiazole,
3-(2,6-difluoro-4-(3-(1-(5-pentylpyrimidin-2-yl)piperidin-4-yl)propoxy)phenyl)-5-isopropyl-1,2,4-oxadiazole,
3-(2,6-difluoro-4-(3-(1-(5-(trifluoromethyl)pyrimidin-2-yl)piperidin-4-yl)propoxy)phenyl)-5-isopropyl-1,2,4-oxadiazole,
3-(2,6-difluoro-4-(3-(1-(5-methoxypyrimidin-2-yl)piperidin-4-yl)propoxy)phenyl)-5-isopropyl-1,2,4-oxadiazole,
3-(2,6-difluoro-4-(3-(1-(5-isopropoxypyrimidin-2-yl)piperidin-4-yl)propoxy)phenyl)-5-isopropyl-1,2,4-oxadiazole,
3-(4-(3-(1-(5-chloropyrimidin-2-yl)piperidin-4-yl)propoxy)-2,6-difluorophenyl)-5-isopropyl-1,2,4-oxadiazole,
3-(4-(3-(1-(5-bromopyrimidin-2-yl)piperidin-4-yl)propoxy)-2,6-difluorophenyl)-5-isopropyl-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-methyl-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-ethyl-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-isopropyl-1,2,4-oxadiazole,
5-(sec-butyl)-3-(2,6-difluoro-4-(3-(1-(5-(5-isobutyl-1,2,4-oxadiazol-3-yl)pyrimidin-2-yl)piperidin-4-yl)propoxy)phenyl)-1,2,4-oxadiazole,
3-(2,6-difluoro-4-(3-(1-(5-(5-isobutyl-1,2,4-oxadiazol-3-yl)pyrimidin-2-yl)piperidin-4-yl)propoxy)phenyl)-5-(methoxymethyl)-1,2,4-oxadiazole,
(S)-1-(3-(2,6-difluoro-4-(3-(1-(5-(5-isobutyl-1,2,4-oxadiazol-3-yl)pyrimidin-2-yl)piperidin-4-yl)propoxy)phenyl)-1,2,4-oxadiazol-5-yl)propan-1-ol,
2-(3-(2,6-difluoro-4-(3-(1-(5-(5-isobutyl-1,2,4-oxadiazol-3-yl)pyrimidin-2-yl)piperidin-4-yl)propoxy)phenyl)-1,2,4-oxadiazol-5-yl)-2-methylpropan-1-ol,
3-(4-(3-(1-(5-chloropyrazin-2-yl)piperidin-4-yl)propoxy)-2,6-difluorophenyl)-5-isopropyl-1,2,4-oxadiazole,
3-(2,6-difluoro-4-(3-(1-(5-(trifluoromethyl)pyridin-2-yl)piperidin-4-yl)propoxy)phenyl)-5-isopropyl-1,2,4-oxadiazole,
3-(2,6-difluoro-4-(3-(1-(3-isopropyl-1,2,4-oxadiazol-5-yl)piperidin-4-yl)propoxy)phenyl)-5-methyl-1,2,4-oxadiazole,
3-(2,6-difluoro-4-(3-(1-(3-isopropyl-1,2,4-oxadiazol-5-yl)piperidin-4-yl)propoxy)phenyl)-5-isopropyl-1,2,4-oxadiazole,
(3-(2,6-difluoro-4-(3-(1-(3-isopropyl-1,2,4-oxadiazol-5-yl)piperidin-4-yl)propoxy)phenyl)-1,2,4-oxadiazol-5-yl)methanol,
2-(4-(3-(1-(5-ethylpyrimidin-2-yl)piperidin-4-yl)propoxy)-2,6-difluorophenyl)-5-methyl-1,3,4-oxadiazole,
2-ethyl-5-(4-(3-(1-(5-ethylpyrimidin-2-yl)piperidin-4-yl)propoxy)-2,6-difluorophenyl)-1,3,4-oxadiazole,
2-(4-(3-(1-(5-ethylpyrimidin-2-yl)piperidin-4-yl)propoxy)-2,6-difluorophenyl)-5-isopropyl-1,3,4-oxadiazole,
5-(4-(3-(1-(5-ethylpyrimidin-2-yl)piperidin-4-yl)propoxy)-2,6-difluorophenyl)-N-isopropyl-1,3,4-oxadiazol-2-amine,
2-(2,6-difluoro-4-(3-(1-(5-(trifluoromethyl)pyrimidin-2-yl)piperidin-4-yl)propoxy)phenyl)-5-methyl-1,3,4-oxadiazole,
2-(2,6-difluoro-4-(3-(1-(5-(trifluoromethyl)pyrimidin-2-yl)piperidin-4-yl)propoxy)phenyl)-5-ethyl-1,3,4-oxadiazole,
2-(2,6-difluoro-4-(3-(1-(5-(trifluoromethyl)pyrimidin-2-yl)piperidin-4-yl)propoxy)phenyl)-5-isopropyl-1,3,4-oxadiazole,
2-(4-(3-(1-(5-chloropyrazin-2-yl)piperidin-4-yl)propoxy)-2,6-difluorophenyl)-5-methyl-1,3,4-oxadiazole,
2-(4-(3-(1-(5-chloropyrazin-2-yl)piperidin-4-yl)propoxy)-2,6-difluorophenyl)-5-ethyl-1,3,4-oxadiazole,
2-(4-(3-(1-(5-chloropyrazin-2-yl)piperidin-4-yl)propoxy)-2,6-difluorophenyl)-5-isopropyl-1,3,4-oxadiazole,
5-(4-(3-(3,5-difluoro-4-(5-methyl-1,3,4-oxadiazol-2-yl)phenoxy)propyl)piperidin-1-yl)-3-propyl-1,2,4-oxadiazole,
5-(4-(3-(3,5-difluoro-4-(5-ethyl-1,3,4-oxadiazol-2-yl)phenoxy)propyl)piperidin-1-yl)-3-propyl-1,2,4-oxadiazole,
5-(4-(3-(3,5-difluoro-4-(5-isopropyl-1,3,4-oxadiazol-2-yl)phenoxy)propyl)piperidin-1-yl)-3-propyl-1,2,4-oxadiazole,
5-(4-(3-(3,5-difluoro-4-(5-methyl-1,3,4-oxadiazol-2-yl)phenoxy)propyl)piperidin-1-yl)-3-isopropyl-1,2,4-oxadiazole,
5-(4-(3-(4-(5-ethyl-1,3,4-oxadiazol-2-yl)-3,5-difluorophenoxy)propyl)piperidin-1-yl)-3-isopropyl-1,2,4-oxadiazole,
5-(4-(3-(3,5-difluoro-4-(5-isopropyl-1,3,4-oxadiazol-2-yl)phenoxy)propyl)piperidin-1-yl)-3-isopropyl-1,2,4-oxadiazole,
5-(4-(3-(3,5-difluoro-4-(5-methyl-1,3,4-oxadiazol-2-yl)phenoxy)propyl)piperidin-1-yl)-3-(2,2,2-trifluoroethyl)-1,2,4-oxadiazole,
3-(4-(3-(3,5-difluoro-4-(5-isopropyl-1,3,4-oxadiazol-2-yl)phenoxy)propyl)piperidin-1-yl)-5-isopropyl-1,2,4-oxadiazole,
2-(4-(3-(1-(5-ethylpyrimidin-2-yl)piperidin-4-yl)propoxy)-2,6-difluorophenyl)-5-isopropyl-1,3,4-thiadiazole,
2-(2,6-difluoro-4-(3-(1-(5-propylpyrimidin-2-yl)piperidin-4-yl)propoxy)phenyl)-5-isopropyl-1,3,4-thiadiazole,
2-(2,6-difluoro-4-(3-(1-(5-pentylpyrimidin-2-yl)piperidin-4-yl)propoxy)phenyl)-5-isopropyl-1,3,4-thiadiazole,
2-(2,6-difluoro-4-(3-(1-(5-fluoropyrimidin-2-yl)piperidin-4-yl)propoxy)phenyl)-5-isopropyl-1,3,4-thiadiazole,
2-(2,6-difluoro-4-(3-(1-(5-(trifluoromethyl)pyrimidin-2-yl)piperidin-4-yl)propoxy)phenyl)-5-isopropyl-1,3,4-thiadiazole,
2-(2,6-difluoro-4-(3-(1-(5-(trifluoromethyl)pyridin-2-yl)piperidin-4-yl)propoxy)phenyl)-5-isopropyl-1,3,4-thiadiazole, and
4-ethyl-2-(4-(3-(1-(5-ethylpyrimidin-2-yl)piperidin-4-yl)propoxy)-2,6-difluorophenyl)thiazole.

In one embodiment, the compound represented by above Formula 1 may be 3-(4-(3-(1-(5-ethylpyrimidin-2-yl)piperidin-4-yl)propoxy)-2,6-difluorophenyl)-5-isopropyl-1,2,4-oxadiazole (hereinafter, compound 1).

In one embodiment, the pharmaceutical composition may include the compound represented by above Formula 1; and at least one of the DPPIV inhibitor, the PPAR agonist, the SGLT2 inhibitor, the insulin secretagogue, the biguanidine drug and the alpha-glucosidase inhibitor.

The DPPIV inhibitor may be at least one of sitagliptin, vildagliptin, saxagliptin, linagliptin, gemigliptin, anagliptin, teneligliptin, alogliptin, trelagliptin, omarigliptin, evogliptin, gosogliptin and dutogliptin.

The PPAR agonist may be at least one of troglitazone, ciglitazone, rosiglitazone, pioglitazone, englitazone, elafibranor, saroglitazar and lobeglitazone.

The SGLT2 inhibitor may be at least one of canagliflozin, dapagliflozin, empagliflozin, ertugliflozin, ipragliflozin, luseogliflozin, remogliflozin, sergliflozin, sotagliflozin and tofogliflozin.

The insulin secretagogue may be at least one of glibenclamide, glipizide, gliclazide, glimepiride, tolazamide, tolbutamide, acetohexamide, carbutamide, chlorpropamide, glibornuride, gliquidone, glisentide, glisolamide, glisoxepide, glyclopyamide, glycylamide, glipentide, repaglinide and nateglinide.

The biguanidine drug may be at least one of metformin, buformin and phenformin. The alpha-glucosidase inhibitor maybe at least one of acarbose, voglibose, emiglitate and miglitol.

In one embodiment, the pharmaceutical composition may include the compound represented by above Formula 1; and at least one of evogliptin, sitagliptin, elafibranor, dapagliflozin, glimepiride, metformin and voglibose.

In one embodiment, the pharmaceutical composition may include above compound 1; and at least one of the DPPIV inhibitor, the PPAR agonist, the SGLT2 inhibitor, the insulin secretagogue, the biguanidine drug and the alpha-glucosidase inhibitor.

In one embodiment, the pharmaceutical composition may include above compound 1; and at least one of evogliptin, sitagliptin, elafibranor, dapagliflozin, glimepiride, metformin and voglibose. In one embodiment, the metabolic diseases may be at least one of obesity and dyslipidemia.

When it is described in the present specification that the pharmaceutical composition includes A and B, the pharmaceutical composition may be construed to mean a composition including A and B; a combination including A and B as a separate preparation, respectively; or a composition including the combination. Thus, in the present invention, the composition may be used interchangeably with the combination.

The pharmaceutical composition may be one which includes a compound represented by Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor; as a separate preparation respectively, or includes all in the form of a combination preparation.

The pharmaceutical composition may be a combination of separate preparations, or a combination preparation.

The pharmaceutical composition may include a first compartment containing a first active ingredient, and a second compartment containing a second active ingredient.

The first active ingredient may be a compound represented by Formula 1, pharmaceutically acceptable salts thereof, optical isomers thereof, hydrates thereof, solvates thereof or mixtures thereof. The second active ingredient may include at least one selected from a DPPIV inhibitor, a PPAR agonist, a SGLT2 inhibitor, an insulin secretagogue, a biguanidine drug and an alpha-glucosidase inhibitor.

According to embodiments of the present invention, in the pharmaceutical composition, the first compartment containing the first active ingredient and the second compartment containing the second active ingredient maybe co-administered, and thus the pharmaceutical composition may be a composition for co-administration. Specifically, the pharmaceutical composition may be a combination in which two ingredients are formulated into separate unit dosage forms. In this case, the first active ingredient and the second active ingredient may be co-administered as separate dosage forms.

In one embodiment of the present invention, it was found that the pharmaceutical composition of one embodiment is effective in the treatment and prevention of diabetes by measuring a blood concentration of active GLP-1 and a concentration of blood glucose through an evaluation of medicinal efficacy of glucose tolerance improvement when single oral administration of the drug(s) is given to normal mice model.

In one embodiment of the present invention, it was found that the pharmaceutical composition of one embodiment is effective in the treatment and prevention of diabetes and metabolic diseases associated therewith through measurement of blood glucose, triglyceride and weight in a mouse model with hyperglycemia, insulin resistance or obesity induced by supplying a special diet.

More specifically, when administering the pharmaceutical composition according to one embodiment, it is confirmed that a blood GLP-1 concentration rises, blood glucose decreases, a plasma triglyceride concentration decreases, and a body weight decreases compared to when co-administering a compound represented by Formula 1, pharmaceutically acceptable salts thereof, optical isomers thereof, hydrates thereof, solvates thereof or mixtures thereof as a GPR119 agonist; or a DPPIV inhibitor, a PPAR agonist, a SGLT2 inhibitor, an insulin secretagogue, a biguanidine drug and an alpha-glucosidase inhibitor, respectively.

In the present invention, a non-limiting example of the pharmaceutically acceptable salt of the compound represented by above Formula 1 may include inorganic acid salts such as hydrochloric acid, bromic acid, phosphoric acid or sulfuric acid; organic carboxylic acid salts such as acetic acid, trifluoroacetic acid, citric acid, maleic acid, oxalic acid, succinic acid, benzoic acid, tartaric acid, fumaric acid, mandelic acid, ascorbic acid or malic acid, or sulfonic acid salts such as methanesulfonic acid or para-toluenesulfonic acid; alkali metal salts such as sodium, potassium or lithium; various acid salts known to be capable of forming other pharmaceutically acceptable salts; or the like.

The pharmaceutical composition for preventing or treating diabetes or at least one disease selected from metabolic diseases associated therewith according to one embodiment may be used in the form of a general pharmaceutical preparation. The pharmaceutical preparation may be administered in various oral and parenteral dosage forms upon administration, and the dosage form may be variously determined according to the method of use.

If the pharmaceutical composition of one embodiment is formulated into several oral and parenteral dosage forms, the composition may be prepared by using the generally used excipients such as fillers, diluents, extenders, binders, humectants, disintegrants, surfactants, etc.

A solid preparation for oral administration may include tablets, pills, powders, granules, capsules, etc., and these solid preparations may be prepared by mixing at least one excipient, for example, starch, calcium carbonate, sucrose, lactose, gelatin or the like in the pharmaceutical composition. In addition, lubricants such as magnesium stearate, talc, etc., may be used in addition to simple excipients.

Furthermore, a liquid preparation for oral administration may include suspending agents, liquids for internal use, emulsions, syrups, etc., but may also include various excipients, for example, humectants, sweetening agents, flavoring agents, preservatives, etc. in addition to water and liquid paraffin, which are the frequently used simple diluents.

A preparation for parenteral administration may include sterile aqueous solutions, non-aqueous solvents, suspensions, emulsions, lyophilized preparations and suppositories. The non-aqueous solvent and the suspending agent used herein may include propylene glycol, polyethylene glycol, vegetable oil like olive oil, injectable ester like ethyl oleate, etc. A base of the suppository used herein may include witepsol, macrogol, tween 61, cacao butter, laurinum, glycerogelatin, etc.

In addition, the pharmaceutical composition for prevention or treatment of diabetes or at least one disease selected from metabolic diseases associated therewith may have an effective amount represented in an administration range of about 1 to about 1,000 mg. An administered amount or an intake amount may be administered as a variety of administered doses and methods, such as once a day or several times a day by dividing the composition depending on a subject's body weight, age, gender, health condition, diet, administration time, administration method, excretion rate, and severity of the disease.

In the present invention, diabetes may include both type 1 diabetes and type 2 diabetes. Type 1 diabetes may refer to a disease characterized by destruction of pancreatic beta cells and an absolute deficiency of insulin, which are developed by the interaction of genetic, environmental, and immunological causes. Type 2 diabetes may refer to a disease which begins with insulin resistance in which cells do not properly respond to insulin, and may develop due to excessive weight and low activity. In type 2 diabetes, insulin deficiency may occur as the disease progresses.

The pharmaceutical composition of one embodiment may include a compound represented by Formula 1 or an active ingredient having a similar function thereto; and at least one selected from an insulin secretagogue, a DPPIV inhibitor, a PPAR agonist, a SGLT2 inhibitor, a biguanidine drug and an alpha-glucosidase inhibitor.

The present invention may also provide a method for preventing or treating diabetes or at least one diseases selected from metabolic diseases associated therewith, including administering into a subject in need of treatment a therapeutically effective amount of a pharmaceutical composition including a compound represented by Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor.

In the present specification, the term "metabolic diseases associated therewith" may refer to a disease which shares some etiological causes with diabetes, such as obesity or dyslipidemia, and is likely to easily occur with the onset of diabetes.

In the present specification, the term "at least one" may be construed to indicate all combinations derived from a plurality of configurations. For example, "at least one of A, B and C" may be construed to include "A, B or C", "two selected from A, B and C" and all combinations derived from "A, B and C."

In the present specification, the term "subject in need of treatment" may refer to mammals including humans, and the term "administration" may mean providing a predetermined material to subjects by any appropriate method. The term "therapeutically effective amount" may refer to an amount of an active ingredient or a pharmaceutical composition which induces animals or humans to show the biological or medical responses considered by investigators, veterinarians, doctors or clinicians, which may include an amount thereof for inducing a relief of symptoms of diseases or disorders to be treated. It is apparent to those skilled in the art that the therapeutically effective dosage and the number of administration for active ingredient of the present invention may vary depending on a desired effect.

In one embodiment, a route of administration of the pharmaceutical composition of the present invention may be administered through any general route as long as it can reach a target tissue.

The pharmaceutical composition of one embodiment may be administered orally, intraperitoneally, intravenously, intramuscularly, subcutaneously, endothelially, intranasally, intrapulmonarily, rectally, intracavitarily, intraperitoneally and intrathecally, but is not limited thereto.

The pharmaceutical composition of one embodiment may be administered once a day or at least twice a day at a certain interval of time.

The pharmaceutical composition of one embodiment may be used alone or in combination with a surgery, endocrinotherapy, drug treatment and methods of using a biologic response modifier, in order to prevent and treat diabetes or at least one disease selected from metabolic diseases associated therewith.

One embodiment may also provide a food composition, for preventing or alleviating diabetes or at least one disease selected from metabolic diseases associated therewith, including a compound represented by above Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor; as an active ingredient.

In one embodiment, the term "alleviation" may refer to all the acts, in which a disease gets better or takes a favorable turn by administering the composition.

In one embodiment, the term "food" may refer to meats, sausages, breads, chocolates, candies, snacks, confectioneries, pizzas, instant noodles, other noodles, chewing gums, dairy products including ice creams, various types of soup, beverages, teas, health drinks, alcohol beverages, vitamin complexes, health functional foods, health foods, health supplement foods and the like, and include all the foods in a conventional sense.

The term "health functional food" may be the same term as food for special health use (FoSHU), and refer to the food having a high medical and medicinal effect, which is processed to efficiently show a biomodulation function in addition to supplying nutrients. In this case, the "function(ality)" may refer to controlling nutrients or obtaining a beneficial effect on health uses such as a physiological action, etc., with regard to the structure and function of the human body.

The "health food" may refer to food having an effect of actively maintaining or enhancing health compared to general food, and "health supplement food" may refer to food for a health supplement purpose. In some cases, the terms of health functional food, health food, and health supplement food may be used interchangeably.

The food of the present invention may be prepared by a method conventionally used in the art, and raw materials and ingredients conventionally added in the art may be added to prepare the food during the preparation. Specifically, proteins, carbohydrates, fats, nutrients, seasoning agents, and flavoring agents may be included, and an example of said carbohydrates may include glucose, fructose, maltose, sucrose, oligosaccharide, dextrin, cyclodextrin, xylitol, sorbitol, erythrol, saccharin or synthetic flavoring agents, but is not limited thereto. The food composition of the present invention may be prepared into various dosage forms without limitation, as long as the dosage form is recognized as food.

The present invention may also provide a method for preventing or alleviating diabetes or at least one disease selected from metabolic diseases associated therewith including administering into a subject in need of alleviation a food composition including a compound represented by above Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor; as an active ingredient.

The present invention may also provide a feed composition for preventing or alleviating diabetes or at least one disease selected from metabolic diseases associated therewith including a compound represented by above Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor; as an active ingredient.

In the present invention, the term "feed" may refer to any natural or artificial diet, one meal, etc., or an ingredient of said one meal, which is to be consumed or digested by livestock or appropriate thereto. The feed may include feed additives or auxiliary feeds.

The kind of said feed is not particularly limited, and the feed conventionally used in the art may be used. Anon-limiting example of said feed may include vegetable feeds such as grains, root fruits, food processing by-products, algae, fibers, pharmaceutical by-products, oils and fats, starches, oil meals, grain by-products or the like; animal feeds such as proteins, inorganic matters, oils and fats, minerals, single-cell proteins, zooplanktons, foods; or the like. The feed may be used alone or by mixing at least two thereof.

The present invention may also provide a use of the pharmaceutical composition including a compound represented by above Formula 1, pharmaceutically acceptable salts thereof, optical isomers thereof, hydrates thereof solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor; as an active ingredient for preventing or treating diabetes or at least one disease selected from metabolic diseases associated therewith.

The present invention may also provide a use of the pharmaceutical composition including a compound represented by above Formula 1, pharmaceutically acceptable salts thereof, optical isomers thereof, hydrates thereof; solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor; as an active ingredient, in the manufacture of a pharmaceutical preparation for preventing or treating of diabetes or at least one disease selected from metabolic diseases associated therewith.

The present invention may also provide a method for preventing or treating diabetes or at least one disease selected from metabolic diseases associated therewith, including administering into a subject in need of treatment a therapeutically effective amount of a pharmaceutical composition including a compound represented by above Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor.

The present invention may also provide a combination of a compound represented by above Formula 1, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor.

The present invention may also provide a pharmaceutical composition for preventing or treating diabetes or at least one disease selected from metabolic diseases associated therewith, including a compound represented by above Formula 1, pharmaceutically acceptable salts thereof, optical isomers thereof, hydrates thereof, solvates thereof or mixtures thereof in combination with at least one selected from a DPPIV inhibitor, a PPAR agonist, a SGLT2 inhibitor, an insulin secretagogue, a biguanidine drug and an alpha-glucosidase inhibitor.

In the therapeutic method, the food composition, the alleviation method, the feed composition, the use and the combination, the compound represented by above Formula 1 may be specifically 3-(4-(3-(1-(5-ethylpyrimidin-2-yl)piperidin-4-yl)propoxy)-2,6-difluorophenyl)-5-isopropyl-1,2,4-oxadiazole.

Matters mentioned in each of the pharmaceutical composition, the therapeutic method, the food composition, the alleviation method, the feed composition, the use and combination according to an embodiment of the present invention are applied the same to each of embodiments, if not contradictory to each other.

### Advantageous Effects

A pharmaceutical composition according to the present invention can alleviate postprandial and fasting blood glucoses when administered into a subject.

The pharmaceutical composition according to the present invention can exhibit an effect of alleviating a blood lipid concentration and reducing body fat when administered into a subject.

Thus, the pharmaceutical composition according to the present invention may be advantageously used for preventing or treating diabetes or metabolic diseases associated therewith.

### Brief Description of the Drawings

FIG. 1 is a graph of showing an effect of a drug(s) on increasing a peak blood concentration of active GLP-1 in five minutes after glucose loading and reducing postprandial blood glucose level for two hours after glucose loading when single oral administration of the drug(s) is given to normal mice.
FIG. 2 is a graph of showing an efficacy of a drug(s) on postprandial blood glucose for two hours after glucose loading when single oral administration of the drug(s) is given to normal mice.
FIG. 3 is a graph of showing an efficacy of co-administration of drugs on nonfasting and fasting blood glucose levels when the drugs are co-administered to HF/STZ diabetic mice for ten weeks.
FIG. 4 is a graph of showing an efficacy of co-administration of drugs on plasma triglyceride concentrations when the drugs are co-administered to HF/STZ diabetic mice for ten weeks.
FIG. 5 is a graph of showing an efficacy of a drug(s) on postprandial blood glucose levels for two hours after glucose loading when single oral administration of the drug(s) is given to normal mice.
FIG. 6 is a graph of showing nonfasting blood glucose levels when each of the drugs is administered alone and co-administered to DIO mice for 12 weeks.
FIG. 7 is a graph of showing levels of plasma concentration of total GLP-1 when each of drugs is administered alone and co-administered to HF mice for three weeks.
FIG. 8 is a graph of showing fasting blood glucose levels when each of the drugs is administered alone and co-administered to HF mice for three weeks.
FIG. 9 is a graph of showing levels of change in body weight loss rate over time and change in body composition improvement when each of the drugs is administered alone and co-administered to HF mice for three weeks.
FIG. 10 is a graph showing an efficacy of drug administration alone and in combination on postprandial blood glucose levels for two hours after sucrose loading when single oral administration of the drug(s) is given to normal mice.

### Mode for Invention

The features and advantages of the present invention as well as methods for achieving them will be apparent with reference to exemplary embodiments described in detail hereinafter. However, the present invention is not limited to the exemplary embodiments disclosed hereinafter, but will be implemented in various different forms. Hereinafter, the following exemplary embodiments will be suggested for better understanding of the present invention and are provided only for the purpose of completely illustrating the scope of the present invention to those skilled in the art, and thus the present invention will be defined only by the scope of the claims thereto.

A pharmaceutical composition for preventing or treating of diabetes or at least one disease selected from metabolic diseases associated therewith according to the present invention may include a compound represented by Formula 1 described above, pharmaceutically acceptable salts thereof, optical isomers thereof, hydrates thereof, solvates thereof or mixtures thereof; and at least one selected from a DPPIV inhibitor, a PPAR agonist, a SGLT2 inhibitor, an insulin secretagogue, a biguanidine drug and an alpha-glucosidase inhibitor.

The compound represented by Formula 1 may be a GPR119 ligand, and G protein-coupled receptor 119 (GPR119) may be one of G protein-coupled receptors (GPCRs) belonging to class A.

GRP119 may be activated by the GRP119 ligand to generate cAMP through Gas among G protein subtypes, thereby inducing intracellular signaling.

GRP119 may have a high expression level in pancreatic beta cells and L cells of small intestines. Thus, when GPR119 of the pancreas is activated by administering the pharmaceutical composition of one embodiment, insulin secretion from pancreatic beta cells may increase, thereby reducing postprandial blood glucose. Thus, when GPR119 is activated, an effect of reducing blood lipids and body weights may be achieved in addition to glycemic control, thus contributing to alleviation of diabetes as well as metabolic diseases associated therewith.

Meanwhile, when GPR119 of small intestines is activated, the secretion of glucagon-like peptide-i (GLP-1) from the small intestine L-cells may be increased. GLP-1 may exhibit a local action of inhibiting an influx of postprandial fat into bloodstream in small intestines.

In general, a GLP-1 receptor-like action may be modulated by high molecular compounds such as peptide ligands. However, since the pharmaceutical composition according to one embodiment includes the GPR119 ligand, which is a relatively low molecular compound, a GLP-1 receptor-like action may be implemented by an oral low molecular compound.

In the compound represented by Formula 1 in one embodiment, a halogen element may be substituted at positions 2 and 6 of a phenyl moiety of 4-(3-phenoxypropyl)piperidine. Accordingly, it may react more sensitively with GPR119 and a degree of GPR119 activation may be sharply higher than when other GPR119 ligands are administered.

The pharmaceutical composition of one embodiment may further include at least one selected from a DPPIV inhibitor, a PPAR agonist, a SGLT2 inhibitor, an insulin secretagogue, a biguanidine drug and an alpha-glucosidase inhibitor, in addition to the compound represented by Formula 1 described above. Thus, the pharmaceutical composition of one embodiment may be administered at least two drugs having different mechanisms, so as to reduce blood glucose through various mechanisms, thereby effectively controlling blood glucose.

Hypoglycemic agents currently applied to diabetic patients may include dipeptidyl peptidase IV (DPPIV) inhibitor, insulin sensitizer, sodium glucose co-transporter 2 (SGLT2) inhibitor for promoting urinary glucose excretion through a mechanism of inhibiting sodium-glucose co-transporter 2, insulin secretagogue, biguanidine drug (Biguanide), alpha-glucosidase inhibitor, etc.

Dipeptidyl peptidase IV (DPPIV) may be an enzyme which inactivates GLP-1 by removing two amino acids from an N-terminus of GLP-1 in the blood. GLP-1, of which secretion is induced by GPR119 ligand, may be inactivated by DPPIV enzyme within minutes after translocation into blood. Thus, when the GPR119 ligand increasing GLP-1 secretion and the DPPIV inhibitor blocking GLP-1 inactivation are used in combination, a duration of action of active GLP-1 in the blood may be extended along with a direct insulin secretion effect according to GPR119 activation of beta cells. Thus, a medicinal efficacy for alleviating metabolic diseases may be excellent. In other words, the DPPIV inhibitor may promote insulin secretion by prolonging a biological action of endogenous GLP-1, and thus may exhibit a postprandial hypoglycemic effect.

The DPPIV inhibitor may include, for example, at least one of sitagliptin, vildagliptin, saxagliptin, linagliptin, gemigliptin, anagliptin, teneligliptin, alogliptin, trelagliptin, omarigliptin, evogliptin, gosogliptin and dutogliptin.

An insulin sensitizer may be a drug for activating a peroxisome proliferator-activated receptor (PPAR). The PPAR agonist may include, for example, at least one of troglitazone, ciglitazone, rosiglitazone, pioglitazone, englitazone, elafibranor, saroglitazar and lobeglitazone.

Drug sodium glucose co-transporter 2 (SGLT2) inhibitor may promote glucose excretion by inhibiting glucose reabsorption in the kidneys in an insulin-independent manner. In addition, it may be accompanied by weight loss through calorie loss due to glucose excretion. The SGLT2 inhibitor may include, for example, at least one of canagliflozin, dapagliflozin, empagliflozin, ertugliflozin, ipragliflozin, luseogliflozin, remogliflozin, sergliflozin, sotagliflozin and tofogliflozin.

The insulin secretagogue may include a drug of sulfonylureas and non-sulfonylureas. For example, the insulin secretagogue of sulfonylureas may include at least one of glibenclamide (glyburide), glipizide, gliclazide, glimepiride, tolazamide, tolbutamide, acetohexamide, carbutamide, chlorpropamide, glibornuride, gliquidone, glisentide, glisolamide, glisoxepide, glyclopyamide, glycylamide and glipentide. The insulin secretagogue of a non-sulfonylurea structure may include at least one of repaglinide and nateglinide.

The biguanidine drug may inhibit gluconeogenesis in the liver. The biguanidine drug may include, for example, at least one of metformin, buformin and phenformin.

The alpha-glucosidase inhibitor may exhibit an action of delaying absorption of carbohydrates by inhibiting an enzyme activity that breaks down disaccharides into monosaccharides in intestines. The alpha-glucosidase inhibitor may include, for example, at least one of acarbose, voglibose, emiglitate and miglitol.

The pharmaceutical composition of one embodiment may include a complex of ingredients. Thus, when administering the pharmaceutical composition of one embodiment, not only a hypoglycemic effect but also an effect of reducing blood lipids and body weights may be achieved by two drugs having different mechanisms of action from each other.

Thus, it may exhibit an excellent effect of treating or preventing diabetes and metabolic diseases associated therewith compared to the case of administering individual ingredients alone.

Hereinafter, the pharmaceutical composition according to one embodiment of the present invention will be described in detail with reference to Examples and Comparative Examples.

### < Example 1> Confirmation of combination efficacy of GPR119 ligand and DPPIV inhibitor

### Evaluation of efficacy of single dose administration in normal mice

Seven-weeks old ICR male mice fasted for at least 16 hours were administered with each of compound 1, sitagliptin or evogliptin as a DDPIV inhibitor alone, or administered with compound 1 in combination with one of sitagliptin and evogliptin. In both administration alone and co-administration, the mice were orally administered compound 1 at 0.3 mg/kg, sitagliptin at 10 mg/kg, and evogliptin at 0.2 mg/kg. At a time point of 30 minutes after drug administration, a glucose solution was orally loaded at 2 g/kg/10 ml.

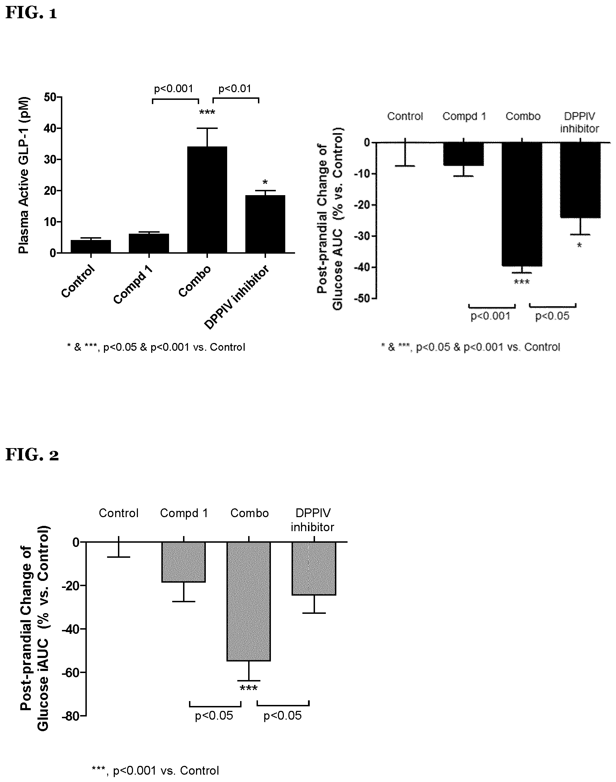

To measure a change in plasma concentrations of active GLP-1 according to the combination, plasma at five minutes after glucose loading was separated using a container pretreated with the DPPIV inhibitor, and the plasma concentrations of active GLP-1 was quantified by using a GLP-1 (Active) ELISA kit (Millipore, Cat. EGLP-35K). To evaluate a drug response to postprandial blood glucose levels, blood glucose levels in whole blood collected from tail vein at a time point of 0, 15, 30, 60, 90, and 120 minutes after glucose challenge were measured using a Roche's AccuChek Active glucose meter. A time-blood glucose level response curve was obtained. In terms of a rate of change, a comparison was made between an area under time-blood glucose level curve in a control group administered with solvent only and an area under the time-blood glucose level curve in a drug administration group. The results thereof are shown in FIG. 1 and FIG. 2.

As confirmed in FIG. 1, when compound 1 and sitagliptin as a DPPIV inhibitor, were administered in combination, the blood concentration of active GLP-1 was significantly increased compared to the group administered compound 1 or sitagliptin alone. In addition, the blood glucose level for two hours after glucose loading was also significantly increased in the co-administration group compared to the group administered compound 1 or sitagliptin alone.

As confirmed in FIG. 2, a synergistic effect of alleviating postprandial blood glucose level was also exhibited when compound 1 and evogliptin as another DPPIV inhibitor.

### Evaluation of efficacy of ten-weeks multiple dose administration in diabetic mice

Seven-weeks old ICR male mice were supplied with a high fat diet (Research Diets Inc., D12492; 60% kcal fat) for three weeks. The mice were divided into groups according to body weight, blood glucose and blood triglyceride concentration at a time point of three weeks after intraperitoneally injecting streptozotocin (STZ) at 80 mg/kg at the third week. Specifically, grouping was performed by selecting individuals of which nonfasting blood glucose level was increased 1SD (standard deviation) or higher and a plasma triglyceride level was increased 2SD or higher compared to normal mice. A drug-diet admixture, in which 100 mg/kg/day of compound 1 and 150 mg/kg/day of sitagliptin were mixed, was given to the selected group. The admixture was supplied for ten weeks. After that, fasting blood glucose levels, nonfasting blood glucose levels and plasma triglyceride concentrations were measured. Fasting and nonfasting blood glucose were measured from blood collected from tail vein using a Roche's AccuChek Active glucose meter. The plasma triglyceride concentration was measured from plasma collected after nonfasting autopsy using an automatic hematology analyzer (Konelab 20i).

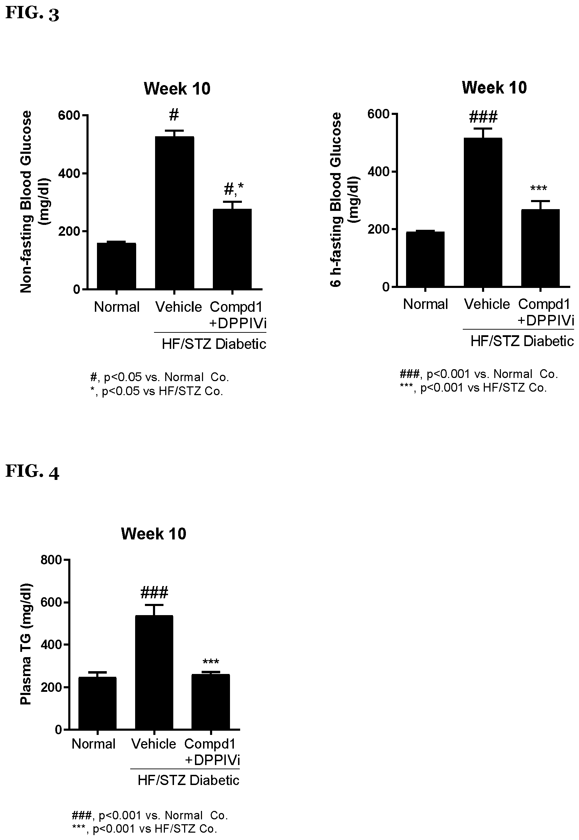

As confirmed in FIG. 3, when compound 1 and sitagliptin were co-administered for ten weeks, it was confirmed that a level of both fasting and nonfasting blood glucose is similar to that of normal mice compared to high fat/streptozotocin (HF/STZ) mice administered with high fat diet only without mixing drugs. In other words, an excellent hypoglycemic rate was exhibited in both fasting and nonfasting blood glucose levels.

As confirmed in FIG. 4, an excellent rate of lowering plasma triglyceride concentration was exhibited in HF/STZ mice with diabetes and hypertriglyceridemia when co-administered with compound 1 and sitagliptin. In particular, when the mice were co-administered with compound 1 and sitagliptin, it is confirmed that the plasma triglyceride concentration is decreased to a level equivalent to that of normal mice, as the plasma triglyceride concentration is about early 200s (mg/dl).

### < Example 2> Confirmation of combination efficacy of GPR119 ligand and SGLT2 inhibitor or insulin secretagogue glimepiride

Seven-weeks old ICR male mice fasted for at least 16 hours were administered with each of compound 1, dapagliflozin as an SGLT inhibitor, or glimepiride as an insulin secretagogue alone, or administered with compound 1 in combination with one of dapagliflozin and glimepiride. Glucose solution was orally loaded at 2 g/kg/10 ml at a time point of 30 minutes after orally administering each of compound 1 at 3 mg/kg, dapagliflozin at 0.3 mg/kg as a SGLT2 inhibitor, or glimepiride at 0.1 mg/kg as an insulin secretagogue alone or in combination. To evaluate a drug response to postprandial blood glucose level, a blood glucose in whole blood collected from tail vein at a time point of 0, 15, 30, 60, 90, and 120 minutes after glucose challenge in the blood was measured with a Roche's AccuChek Active glucose meter. A time-blood glucose level response curve was obtained. In terms of a rate of change, a comparison was made between an area under the time-blood glucose level curve in a control group administered with solvent only and an area under the time-blood glucose level curve in a drug administration group.

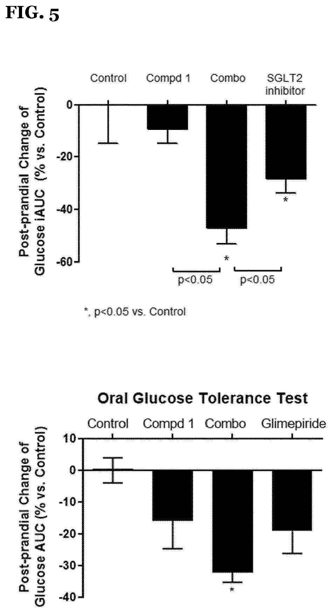

As confirmed in FIG. 5, a hypoglycemic efficacy was increased more significantly when co-administered with the two drugs than when administered alone.

### < Example 3> Confirmation of combination efficacy of GPR119 ligand and PPAR agonist

To induce insulin resistance, six-weeks old male C57BL/6J mice were supplied with a special diet of high fat, high fructose and high cholesterol (Research Diets Inc., D09100301) for at least 27 weeks. A drug-diet admixture was prepared so that diet-induced obese (DIO) and insulin resistant mice could be administered with each of compound 1 at 100 mg/kg/day and elafibranor at 30 mg/kg/day as a PPAR agonist alone or in combination. The prepared drug-diet admixture was supplied to mice for 12 weeks. Nonfasting blood glucose level was measured from plasma collected after autopsy at a time point of 12 weeks after drug administration using a Roche's AccuChek Active glucose meter.

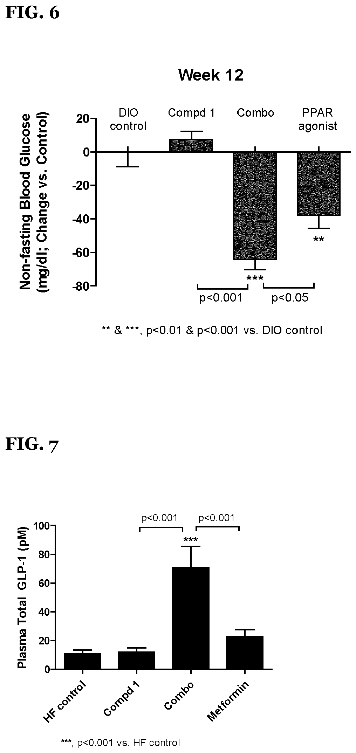

As confirmed in FIG. 6, compound 1 alone did not exhibit a significant hypoglycemic effect in severe insulin resistant mice, and elafibranor alone exhibited a hypoglycemic effect according to the alleviation of insulin resistance. However, when the two drugs were used in combination, it was confirmed that a significantly excellent hypoglycemic effect is exhibited compared to each drug alone.

### < Example 4> Confirmation of combination efficacy of GPR119 ligand and biguanidine drug

Four-weeks old C57BL/6J male mice were supplied with a high fat diet (Research Diets Inc., D12492; 60% kcal fat) for ten weeks so as to induce insulin resistance. After acclimatization to drug administration for two weeks, the mice were assigned into each group based on body weight and body fat. Compound 1 was administered alone at 50 mg/kg twice a day to a compound 1 monotherapy group, and metformin was administered alone at 150 mg/kg twice a day to a metformin monotherapy group. The co-administration group was orally co-administered with compound 1 at 50 mg/kg and metformin at 150 mg/kg twice a day for three weeks. A body weight was measured over time until a time point of administration on day 16, and a body composition was sequentially measured by using a Minispec LF90II NMR spectrometer (Bruker Optics, Ettlingen, Germany) in the last week. Fasting blood glucose was measured from whole blood collected from a tail vein after fasting for six hours at the second week of administration using a Roche's AccuChek Active glucose meter. After administration for three weeks, a nonfasting autopsy was performed and a plasma concentration of total GLP-1 was quantified using the Total GLP-1 ELISA (7-36 and 9-36) Assay Kit (Alpco, 43-GPTHU-E01).

As confirmed in FIG. 7, a significant increase in GLP-1 secretion was not identified in mice administered with compound 1 or metformin alone. However, when co-administered with the two drugs, it was confirmed that a blood concentration of total GLP-1 is significantly increased compared to the administration of each drug alone, thus achieving a synergistic effect of increasing GLP-1 secretion according to the drugs combination.

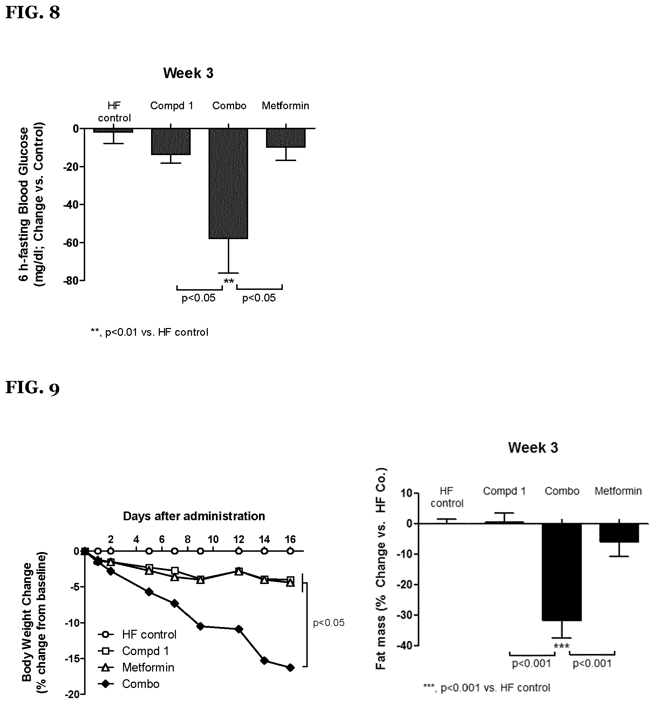

As confirmed in FIG. 8, when the mice were administered with each drug alone, there was a slight tendency to decrease fasting blood glucose levels. However, when the mice were co-administered with the two drugs, a significant fasting hypoglycemic effect was exhibited. In other words, there was a synergistic increase in medicinal efficacy according to the co-administration.

As confirmed in FIG. 9, when the mice were administered with each of compound 1 and metformin alone for two weeks, there was a weight loss of -4.0% and -4.4%, respectively. In contrast, when the mice were co-administered with both drugs for two weeks, there was a weight loss of -16.3%. In other words, when the mice were co-administered with both drugs, it is confirmed that reactivity to the drugs is significantly increased compared to when administered with each drug alone. From this, it was confirmed that co-administration of both drugs has a synergistic effect on the prevention or treatment of obesity.

Referring to the results of body composition analysis performed after the end of administration for three weeks in FIG. 9, there was no effect of reducing body fat when the mice were administered with compound 1 alone for two weeks. When the mice were administered with metformin alone for two weeks, there was an insignificant effect of reducing body fat. When the mice were co-administered with both drugs, however, it was confirmed that there is a rate of decrease in body fat of about 30%. In other words, when the mice were co-administered with both drugs, it was confirmed that there are not only a hypoglycemic effect but also a body fat reduction effect. From this, it was confirmed that co-administration of both drugs has a synergistic effect on the prevention or treatment of obesity.

### < Example 5> Confirmation of combination efficacy of GPR119 ligand and α-glucosidase inhibitor

Eight-weeks old ICR male mice fasted for at least 16 hours were orally administered with each of compound 1 at 3 mg/kg and voglibose at 0.02 mg/kg as an alpha-glucosidase inhibitor alone or in combination. At a time point of 30 minutes later, sucrose solution was orally loaded at 2 g/kg/10 ml. To evaluate a drug response to postprandial blood glucose levels, a blood glucose in whole blood collected from tail vein at a time point of 0, 15, 30, 60, 90, and 120 minutes after glucose injection in the blood was measured with a Roche's AccuChek Active glucose meter. A time-blood glucose level response curve was obtained. In terms of a rate of change, a comparison was made between an area under the time-blood glucose level curve in a control group administered with solvent only and an area under the time-blood glucose level curve in a drug administration group.

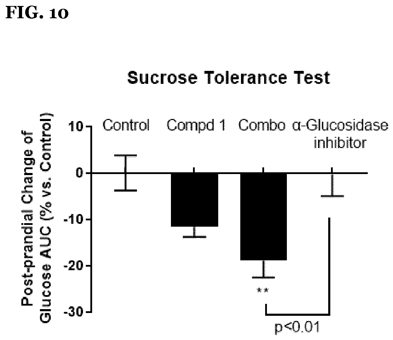

As confirmed in FIG. 10, when the mice were administered with voglibose alone, there was no hypoglycemic effect. However, when the mice were co-administered with compound 1 and voglibose, there was a synergistic effect of increasing a hypoglycemic efficacy more than when administered with compound 1 alone.

## Claims

1. A pharmaceutical composition, for preventing or treating diabetes or at least one disease selected from metabolic diseases associated therewith, comprising a compound represented by a following chemical Formula 1, pharmaceutically acceptable salts thereof, optical isomers thereof, hydrates thereof, solvates thereof, or mixtures thereof; and at least one selected from a DPPIV inhibitor, a PPAR agonist, a SGLT2 inhibitor, an insulin secretagogue, a biguanidine drug and an alpha-glucosidase inhibitor: 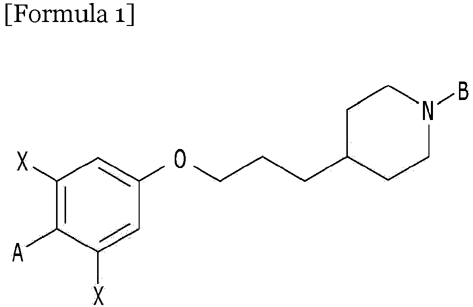 wherein in above Formula 1,
A is an oxadiazole group, a dihydrooxazole group, a thiazole group or a thiadiazole group, in which above A is unsubstituted or substituted with at least one substituent selected from the group consisting of a halogen atom, a C1-C6 straight or branched chain alkyl group, and a C1-C6 straight or branched chain hydroxyalkyl group, in which the alkyl group or the hydroxyalkyl group is each independently unsubstituted or substituted with a halogen atom or a C1-C6 alkoxy group;
B is a pyridine group, a pyrimidine group, a pyrazine group or an oxadiazole group, in which above B is unsubstituted or substituted with at least one substituent selected from the group consisting of a halogen atom, a C1-C6 straight or branched chain alkyl group, a C1-C6 straight or branched chain hydroxyalkyl group, a C1-C6 alkoxy group and an oxadiazole group, in which the C1-C6 straight or branched chain alkyl group, the C1-C6 straight or branched chain hydroxyalkyl group, the C1-C6 alkoxy group or the oxadiazole group is each independently unsubstituted or substituted with halogen, a C1-C6 alkyl group or a C1-C6 alkoxy group; and
X₁ and X2 are each independently F, Cl, Br or I.

2. The pharmaceutical composition according to claim 1, wherein above A is 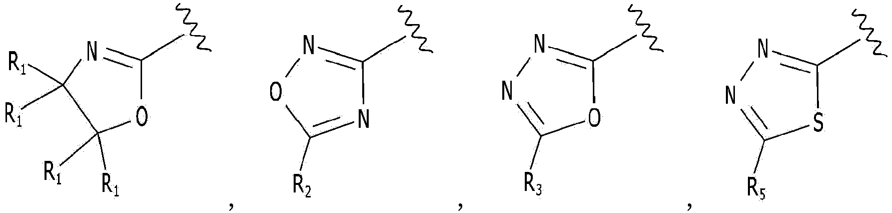 or 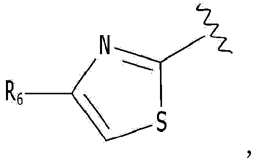 and
R₁ to R₃, R₅ and R₆ are each independently at least one substituent selected from the group consisting of a hydrogen atom, a halogen atom, a C1-C6 straight or branched chain alkyl group and a C1-C6 straight or branched chain hydroxyalkyl group, in which the alkyl group or the hydroxyalkyl group is each independently unsubstituted or substituted with a halogen atom or a C1-C6 alkoxy group.

3. The pharmaceutical composition according to claim 1, wherein above B is 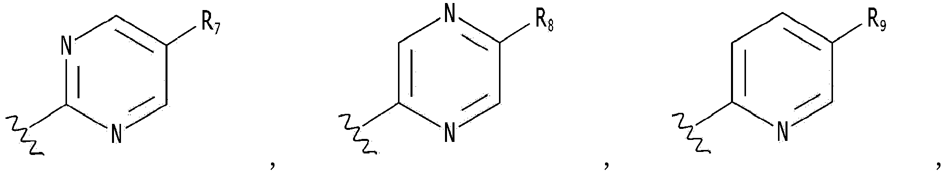 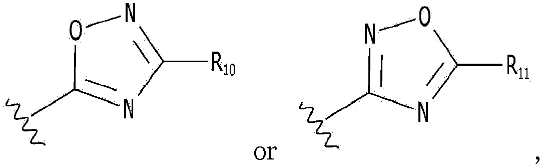 and
R₇ to R₁₁ are each independently substituted with at least one substituent selected from the group consisting of a hydrogen atom, a halogen atom, a C1-C6 straight or branched chain alkyl group, a C1-C6 straight or branched chain hydroxyalkyl group, a C1-C6 alkoxy group and an oxadiazole group, in which the alkyl group, the hydroxyalkyl group, the alkoxy group or the oxadiazole group is each independently unsubstituted or substituted with a halogen atom, a C1-C6 alkyl group or a C1-C6 alkoxy group.

4. The pharmaceutical composition according to claim 1, wherein X is F.

5. The pharmaceutical composition according to claim 1, wherein above A is an oxadiazole group substituted with a C1-C6 straight or branched chain alkyl group, above B is a pyrimidine group substituted with a C1-C6 straight or branched chain alkyl group, and X is F.

6. The pharmaceutical composition according to claim 1, wherein the compound represented by above Formula 1 is 3-(4-(3-(1-(5-ethylpyrimidin-2-yl)piperidin-4-yl)propoxy)-2,6-difluorophenyl)-5-isopropyl-1,2,4-oxadiazole.

7. The pharmaceutical composition according to claim 6, wherein the pharmaceutical composition comprises at least one of the DPPIV inhibitor, the PPAR agonist, the SGLT2 inhibitor, the insulin secretagogue, the biguanidine drug and the alpha-glucosidase inhibitor.

8. The pharmaceutical composition according to claim 6, wherein the pharmaceutical composition comprises at least one of evogliptin, sitagliptin, elafibranor, dapagliflozin, glimepiride, metformin and voglibose.

9. The pharmaceutical composition of claim according to claim 1, wherein the pharmaceutical composition comprises at least one of the DPPIV inhibitor, the PPAR agonist, the SGLT2 inhibitor, the insulin secretagogue, the biguanidine drug and the alpha-glucosidase inhibitor.

10. The pharmaceutical composition according to claim 9, wherein the pharmaceutical composition comprises at least one of evogliptin, sitagliptin, elafibranor, dapagliflozin, glimepiride, metformin and voglibose.

11. The pharmaceutical composition according to claim 1, wherein the metabolic disease is at least one of obesity and dyslipidemia.

12. Use of a pharmaceutical composition, comprising the compound represented by Formula 1 according to claim 1, pharmaceutically acceptable salts thereof, optical isomers thereof, hydrates thereof, solvates thereof, or mixtures thereof; and at least one selected from a DPPIV inhibitor, a PPAR agonist, a SGLT2 inhibitor, an insulin secretagogue, a biguanidine drug and an alpha-glucosidase inhibitor, for preventing or treating diabetes or at least one disease selected from metabolic diseases associated therewith.

13. Use of a pharmaceutical composition, comprising a compound represented by Formula 1 according to claim 1, pharmaceutically acceptable salts thereof, optical isomers thereof, hydrates thereof, solvates thereof, or mixtures thereof; and at least one selected from a DPPIV inhibitor, a PPAR agonist, a SGLT2 inhibitor, an insulin secretagogue, a biguanidine drug and an alpha-glucosidase inhibitor, in the manufacture of a pharmaceutical preparation for preventing or treating diabetes or at least one disease selected from metabolic diseases associated therewith.

14. A method for preventing or treating diabetes or at least one disease selected from metabolic diseases associated therewith comprising administering a therapeutically effective amount of a pharmaceutical composition including a compound represented by Formula 1 according to claim 1, pharmaceutically acceptable salts thereof, optical isomers thereof, hydrates thereof, solvates thereof, or mixtures thereof; and at least one selected from a DPPIV inhibitor, a PPAR agonist, a SGLT2 inhibitor, an insulin secretagogue, a biguanidine drug and an alpha-glucosidase inhibitor, into a subject in need of treatment.
